# EUROPÄISCHE PATENTSCHRIFT

(11) **EP 2 227 157 B1**
(45) Veröffentlichungstag und Bekanntmachung des Hinweises auf die Patenterteilung: **17.07.2013**
(21) Anmeldenummer: 07856762.5
(22) Anmeldetag: 14.12.2007
(51) Int. Cl.: A61B 17/68

(54) **IMPLANTAT ZUR VERWENDUNG FÜR BENACHBART ZUEINANDER ANGEORDNETE KNOCHENPLATTEN**
IMPLANT FOR USE FOR ADJACENTLY ARRANGED BONE PLATES
IMPLANT DESTINÉ À ÊTRE UTILISÉ SUR DES PLAQUES D'OSTÉOSYNTHÈSE DISPOSÉES AU VOISINAGE L'UNE DE L'AUTRE

(43) Veröffentlichungstag der Anmeldung: 15.09.2010
(60) Teilanmeldung: 13000737.0
(73) Patentinhaber: Stryker Leibinger GmbH & Co. KG, 79111 Freiburg (DE)
(72) Erfinder: KNÖPFLE, Christian, 78166 Donaueschingen (DE); MAURER, Regine, 79106 Freiburg (DE); RÜBECAMP, Reinhard, 60437 Frankfurt (DE)
(74) Vertreter: Röthinger, Rainer
(86) Internationale Anmeldenummer: PCT/EP2007/011024
(87) Internationale Veröffentlichungsnummer: WO 2009/076978

(56) Entgegenhaltungen:
- EP-A- 1 192 909
- EP-A- 1 808 140
- EP-A- 1 836 981
- WO-A-2004/028384
- DE-C1- 19 952 359
- DE-U1- 9 490 219
- JP-A- 5 220 174
- JP-A- 2003 180 706
- JP-A- 2003 235 860
- US-A1- 2002 169 455
- US-A1- 2004 034 352

## Beschreibung

### Gebiet der Erfindung

Die Erfindung betrifft ein Implantat beispielsweise zur Festlegung eines Knochendeckels an einem Schädelknochen oder zur Abdeckung von Bohrlöchern im Schädelknochen. Das Implantat besitzt ein erstes Anlageteil, ein zweites Anlageteil sowie ein Kopplungselement zur zugfesten Kopplung des ersten Anlageteils mit dem zweiten Anlageteil derart, dass der Knochendeckel und der Schädelknochen zwischen den beiden Anlageteilen angeordnet sind.

### Hintergrund

Im Vorfeld der Entfernung eines Gehirn-Tumors unterhalb des Schädelknochens muss, um Zugang zum Tumor zu erhalten, der Schädelknochen geöffnet werden. Zu diesem Zweck wird nach dem Einbringen wenigstens eines Bohrlochs in den Schädelknochen ein Knochendeckel aus dem Schädelknochen herausgesägt. Nach der chirurgischen Entfernung des Tumors muss der Schädelknochen wieder verschlossen werden. Dazu wird in der Regel der zuvor ausgesägte Knochendeckel mittels geeigneter Implantate wieder am Schädelknochen festgelegt. Diese Implantate werden häufig auch zur Abdeckung der Bohrlöcher verwendet.

Zum Festlegen eines Knochendeckels an einem Schädelknochen und zur Bohrlochabdeckung sind verschiedene Implantate bekannt. So wird in einer Broschüre der Firma Codman & Shurtieff aus dem Jahr 1965 der sogenannte "Todd Burr Hole Button" beschrieben. Dieses Implantat umfasst ein erstes, scheibenförmiges Anlageteil mit einem von diesem Anlageteil mittig abstehenden Schaft. Ein zweites, ebenfalls scheibenförmiges Anlageteil besitzt eine mittige Öffnung und wird auf den Schaft derart aufgefädelt, dass sich dieser durch die Öffnung hindurch erstreckt.

Der Schaft ist wie die beiden Anlageteile aus einem elastischem Kunststoff-Material gefertigt und besitzt einen Außendurchmesser, der etwas größer ist als der Innendurchmesser der Öffnung des zweiten Anlageteils. Eine auf den Schaft in axialer Richtung wirkende Zugkraft bewirkt daher eine elastische Verlängerung des Schafts und eine mit der Verlängerung einhergehende Verringerung des Schaft-Außendurchmessers. In Folge dieser Verringerung des Außendurchmessers kann das zweite Anlageteil auf den Schaft aufgefädelt und entlang des Schafts in Richtung auf das erste Anlageteil bewegt werden. Wird die auf den Schaft wirkende Zugkraft reduziert, wächst der Außendurchmesser des Schafts wieder an und sorgt für eine zugfeste Kopplung des zweiten Anlageteils mit dem Schaft und somit auch mit dem am Schaftende ausgebildeten ersten Anlageteil.

Zur Befestigung eines Knochendeckels an einem Schädelknochen finden mehrere solche Implantate Verwendung. Dabei wird der als Kopplungselement für die beiden Anlageteile wirkende Schaft in einen Spalt zwischen dem Knochendeckel und dem Schädelknochen derart eingefügt, dass der Knochendeckel und der Schädelknochen zwischen den beiden Anlagenteilen eingeklemmt werden.

Das aus der Broschüre von Codman & Shurtleff bekannte Implantat konnte sich in der Praxis nicht durchsetzen. Ein Grund hierfür liegt in der mühsamen Handhabung des Implantats. So hat sich das Aufbringen einer Zugkraft auf den Schaft sowie das Verschieben eines Anlageteils entlang des Schafts bei aufrecht erhaltener Zugkraft als äußerst umständlich herausgestellt. Außerdem ist das Implantat nicht in der Lage, hohe Klemmkräfte langfristig und zuverlässig aufrecht zu erhalten.

Ein ähnliches Implantat ist aus der EP 1 836 981 A2 bekannt, das ein zweites Anlageteil aufweist, in das eine speziell geformte Beilagscheibe eingesetzt werden kann. Diese Beilagscheibe weist einen konischen Zentralbereich auf, der sich um die mittig angeordnete Öffnung erstreckt, sowie einen flachen Randbereich. Die Beilagscheibe ist in einer Vertiefung an dem zweiten Anlageteil derart aufgenommen, dass sie sich in einer Richtung senkrecht zu einem als Schaft ausgebildeten Kopplungselement an den Seitenwänden der Ausnehmung abstützen kann. Wie bei dem Implantat, das aus der Broschüre von Codman & Shurtleff bekannt ist, ist der Schaft elastisch ausgeführt und der Innendurchmesser der zentralen Öffnung der Beilagscheibe ist etwas kleiner als der Außendurchmesser des Schafts. Die konische Ausbildung des Zentralbereichs der Beilagscheibe vereinfacht zusätzlich das Auffädeln, während die Abstützung der Beilagscheibe an den Seitenwänden der Ausnehmung ein Abflachen des konischen Zentralbereichs verhindert und dadurch entsprechende Klemmkräfte zur Fixierung der Beilagscheibe relativ zu dem Schaft erreicht werden können. Dennoch können sich auch bei diesem Implantat die bereits in dem Zusammenhang mit den aus der Broschüre von Codman & Shurtleff bekannten Implantaten genannten Nachteile einstellen.

Aus der JP 05 220 174 A ist ein weiteres Implantat zur Festlegung eines Knochendeckels an einem Schädelknochen bekannt, das einfacher handhabbar ist. Das Implantat umfasst ein rechteckiges erstes Anlageteil mit einem davon abstehenden Band sowie ein rechteckiges zweites Anlageteil. Das zweite Anlageteil besitzt eine zentrale Öffnung und wird auf das Band derart aufgefädelt, dass sich das Band durch diese Öffnung hindurch erstreckt.

Das Band des Implantats ist ein- oder beidseitig mit Zähnen versehen. Das auf das Band aufgefädelte Auflageteil besitzt bis zu zwei bewegliche Klauen, die sich in Eingriff mit den am Band ausgebildeten Zähnen befinden. Sowohl die Zähne als auch die Klauen weisen eine sägezahnartige Profilierung auf, welche einer Bewegung des aufgefädelten Anlageteils in Richtung auf das andere Anlageteil einen geringeren Widerstand entgegensetzt als einer Bewegung in die umgekehrte Richtung. Dieser Sachverhalt sorgt für eine zugfeste Kopplung der beiden Anlageteile derart, dass zwischen ihnen der Knochendeckel und der Schädelknochen zuverlässig einklemmbar sind.

Aus der JP 2003 235860 A und der JP 2003 180706 A sind zwei Implantate bekannt, bei denen in das zweite Anlageteil ein zusätzliches Element mit einem Festlege-Element und einem Gegenlager zum Aufbringen der notwendigen Klemmkraft zur Festlegung des zweiten Anlageteils eingesetzt ist.

Aus der DE 199 52 359 ist weiterhin ein Implantat zur Festlegung eines Knochendeckels an einem Schädelknochen bekannt, bei dem an einem zweiten Anlageteil eine zentrale Durchgangsöffnung mit vorstehenden Haltelaschen in der Form einer Federzunge ausgebildet ist, wobei die Haltelaschen in einem Winkel zu der Anlagefläche des zweiten Anlageelements abgewandt von dieser vorstehen. Auch bei diesem Implantat werden die Anlageteile mit Hilfe eines bauchförmigen Kopplungselements miteinander verbunden, an dem Zähne ausgebildet sind, in die die Haltelaschen eingreifen können. Durch die Anordnung der Haltelaschen in einem Winkel zu der Anlagefläche wird einer Bewegung des aufgefädelten Anlageteils in Richtung auf das andere Anlageteil ein geringerer Widerstand entgegengesetzt als einer Bewegung in die umgekehrte Richtung. Jedoch können bei einer üblichen Ausbildung der Anlageteile aus Kunststoff diese Haltelaschen in der Praxis abbrechen, wodurch die Funktion des zweiten Anlageteils nicht mehr ausgeführt werden kann.

Schließlich ist aus der DE 94 90 219 A1 eine Sperre-Kompressionsvorrichtung bekannt, die zum Zusammendrücken und -klammern von Knochen genutzt werden kann.

Der Erfindung liegt die Aufgabe zugrunde, ein Implantat zur Festlegung zweier benachbart zueinander angeordneter Knochenplatten anzugeben, das eine verbesserte strukturelle Stabilität bezüglich der im Rahmen der Festlegung des Implantats auftretenden Zug- und Klemmkräfte aufweist.

### Kurzer Abriss der Erfindung

Gemäß einem ersten Aspekt wird die Aufgabe gelöst durch ein Implantat zur Verwendung für benachbart zueinander angeordnete Knochenplatten mit einem ersten Anlageteil, einem zweiten Anlageteil, das einen Grundkörper und einen daran befestigten Einsatz aufweist, wobei wenigstens ein auslenkbares Festlege-Element entweder am Grundkörper oder am Einsatz ausgebildet ist, und mit einem sich durch das zweite Anlageteil hindurch erstreckenden und mit dem Festlege-Element zusammenwirkenden Kopplungselement zur zugfesten Kopplung des ersten Anlageteils mit dem zweiten Anlageteil, wobei das Kopplungselement in einen Spalt zwischen den Knochenplatten derart einfügbar ist, dass die beiden Knochenplatten zwischen den beiden Anlageteilen angeordnet sind. Für das wenigstens eine Festlege-Element ist ein Gegenlager an der anderen Komponente von Einsatz und Grundkörper ausgebildet, so dass das Gegenlager das Festlege-Element bei einer Bewegung des zweiten Anlageteils vom ersten Anlageteil weg abzustützen vermag.

Gemäß einer Weiterbildung besitzt das zweite Anlageteil wenigstens zwei sich bezüglich des Kopplungselements gegenüberliegende Festlege-Elemente, und für jedes Festlege-Element ist ein Gegenlager vorgesehen. So können bei einem bandförmigen Kopplungselement beispielsweise zwei Festlege-Elemente vorgesehen sein, wobei jedes Festlege-Element mit einer der beiden langen Seiten des Bandes zusammenwirkt. Bei einem Verbindungselement mit rundem oder quadratischem Querschnitt können auch drei, vier oder mehr Festlege-Elemente vorhanden sein.

Das wenigstens eine Festlege-Element ist gemäß einer Variante in einer Richtung parallel zu einer Längsachse des Kopplungselements auslenkbar. In diesem Zusammenhang kann das wenigstens eine Festlege-Element als Zunge oder Klaue ausgebildet sein. Eine Zunge kann durch zwei oder mehr Materialschnitte definiert werden.

Gemäß einer Ausgestaltung besitzt das zweite Anlageteil einen Grundkörper und einen daran befestigten Einsatz. Der Grundkörper und der Einsatz können aus gleichen oder aber aus unterschiedlichen Materialien bestehen. Als Materialien kommen insbesondere Kunststoffe und Metalle in Frage.

Bei einem mehrteiligen zweiten Anlageteil kann das wenigstens eine Festlege-Element im Einsatz ausgebildet sein, während das Gegenlager am Grundkörper des zweiten Anlageteils ausgebildet ist. Alternativ hierzu können das Festlege-Element am Grundkörper und das Gegenlager am Einsatz vorgesehen werden. Natürlich kommen für das zweite Anlageteil auch einstückige Ausführungen in Frage, so dass das Festlege-Element und das Gegenlager in ein und dem selben Bauteil realisierbar sind.

Das Kopplungselement kann mit einer Oberflächenkonturierung versehen sein. Das wenigstens eine Festlege-Element kann in diesem Fall zum zugfesten Festlegen des Kopplungselements am zweiten Anlageteil mit der Oberflächenkonturierung zusammenwirken. Bei der Oberflächenkonturierung kann es sich um eine Abfolge von beliebig geformten Zähnen mit symmetrischem oder asymmetrischem Profil handeln. Gemäß einer Variante besitzen die Zähne ein asymmetrisches (z. B. ein sägezahnartiges) Profil derart, dass einer Bewegung des zweiten Anlageteils auf das erste Anlageteil zu ein geringerer Wiederstand entgegengesetzt wird als einer Bewegung in die umgekehrte Richtung.

Weiterhin kann der Einsatz mittels wenigstens eines Verbindungselements mit dem Grundkörper verbunden sein. Das Verbindungselement kann sich dabei bezüglich einer Bewegung in eine Lösestellung des Verbindungselements am Kopplungselement abstützen.

Das wenigstens eine Verbindungselement kann am Grundkörper ausgebildet sein und mit dem Einsatz zusammenwirken oder am Einsatz ausgebildet sein und dann mit dem Grundkörper zusammenwirken. Beide Aspekte lassen sich auch dahingehend kombinieren, dass wenigstens ein Verbindungselement am Grundkörper und wenigstens ein weiteres Verbindungselement am Einsatz vorgesehen sind.

Der Einsatz und der Grundkörper können auf verschiedene Art und Weise mittels des wenigstens einen Verbindungselements miteinander verbunden sein. In diesem Zusammenhang können beispielhaft formflüssige und kraftschlüssige Verbindungen genannt werden. Gemäß einer Variante ist das Verbindungselement als Rastelement ausgebildet. Das Rastelement kann einen Rasthaken aufweisen, welcher sich bezüglich eines Entrastens am Kopplungselement abstützt.

Wie oben bereits erläutert, kann der Einsatz wenigstens ein Festlege-Element aufweisen, das dazu ausgebildet ist, das zweite Anlageteil am Kopplungselement festzulegen. Bei dem Kopplungselement kann es sich um ein Band handeln; es sind jedoch auch stift-schnur- oder drahtförmige Kopplungselemente denkbar. Außerdem können die beiden Anlageteile durch mehr als ein Kopplungselement miteinander gekoppelt werden. Es können beispielsweise zwei oder mehr parallele Bänder oder Stifte vorgesehen sein.

Weiterhin kann vorgesehen sein, dass das wenigstens eine konkav gekrümmte Anlageteil eine Mehrzahl von Rippen aufweist, die sich ausgehend von einem zentralen Abschnitt dieses Anlageteils radial nach außen erstrecken.

Die einzelnen Rippen beginnen vorzugsweise ungefähr in der Mitte des Anlageteils und können sich bis zumindest in die Nähe des Außenumfangs des jeweiligen Anlageteils erstrecken. Die Rippen müssen nicht alle die gleiche Gestalt und Länge aufweisen. So können manche Rippen kürzer und andere Rippen länger ausgebildet sein.

Gemäß einer Option sind zwischen vier und zehn Rippen vorgesehen. Die Rippen können gleichmäßig oder auch ungleichmäßig voneinander beabstandet sein. Jeweils zwei zueinander benachbarte Rippen können jeweils ein Anlageteil-Segment definieren. Sind vier oder mehr derartiger Segmente vorgesehen, wird jedes Segment eine Krümmung im Wesentlichen nur in radialer Richtung aufweisen. Mit anderen Worten, die Krümmung eines Segments in Umfangsrichtung wird in einem solchen Fall deutlich geringer sein als dessen Krümmung in radialer Richtung.

Gemäß einer ersten Variante weisen beide Anlageteile jeweils eine konkave Krümmung und Rippen auf. Gemäß einer zweiten Variante ist lediglich eines der beiden Anlageteile, beispielsweise das zweite, konkav gekrümmt und mit Rippen versehen.

Wie bereits erwähnt, stehen für das Implantat verschiedene Materialien und Materialkombinationen zur Verfügung. Wenigstens eines der beiden Anlageteile kann vollständig aus Kunststoff gefertigt sein. Es ist jedoch auch denkbar, ein aus einem Grundkörper mit Einsatz bestehendes Anlageteil aus einer Metall-Kunststoff Kombination zu fertigen. In einem solchen Fall kann der Grundkörper aus Kunststoff gefertigt sein und der Einsatz aus Metall (oder umgekehrt). Als Kunststoffmaterial kommen beispielsweise bio-absorbierbare und/oder thermoplastische Kunststoffe wie Polyetheretherketon (PEEK) oder dessen Abkömmlinge und Derivate in Frage.

Das erste Anlageteil und das Kopplungselement können einstückig gefertigt sein oder aber als separate Komponenten. Im zuletzt genannten Fall wird das erste Anlageteil erst nach seiner Herstellung mit dem Kopplungselement lose oder starr verbunden.

### Kurze Beschreibung der Zeichnungen

Weitere Merkmale und Vorteile der Erfindung ergeben sich aus der nachfolgenden Beschreibung eines bevorzugten Ausführungsbeispiels. Es zeigen:
- Fig. 1: eine perspektivische Ansicht eines fertig montierten Ausführungsbeispiels eines Implantats;
- Fig. 2: eine Schnittansicht des fertig montierten Implantats gemäß Fig. 1;
- Fig. 3: eine Seitenansicht einer Baugruppe des Implantats gemäß Fig. 1;
- Fig. 4: eine Ansicht der Baugruppe von Fig. 3 von unten;
- Fig. 5: eine perspektivische Ansicht des Grundkörpers des oberen Anlageteils des Implantats gemäß Fig. 1;
- Fig. 6: eine Schnittdarstellung des Grundkörpers gemäß Fig. 5;
- Fig. 7: eine perspektivische Darstellung eines Einsatzes zum Einbau in den Anlageteil-Grundkörper gemäß Fig. 5;
- Fig. 8: eine erste Schnittdarstellung des Einsatzes gemäß Fig. 7; und
- Fig. 9: eine zweite Schnittdarstellung des Einsatzes gemäß Fig. 7

### Beschreibung eines bevorzugten Ausführungsbeispiels

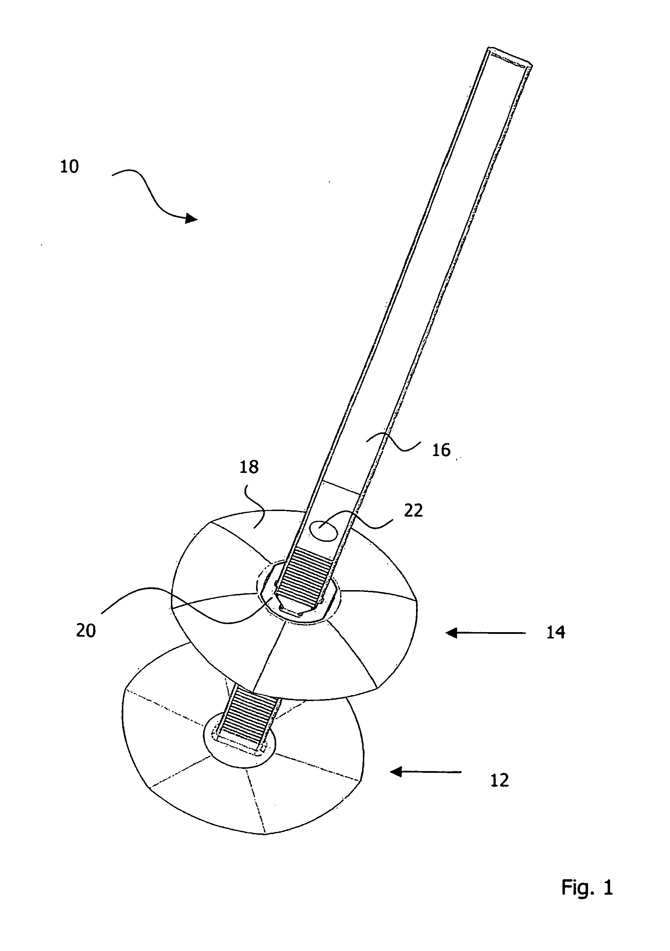

Fig. 1 zeigt eine perspektivische Ansicht eines Ausführungsbeispiels eines Implantats 10 zur Festlegung zweier benachbart zueinander angeordneter Knochenplatten. Das Implantat ist dazu ausgebildet, zur Festlegung eines Knochendeckels an einem Schädelknochen verwendet zu werden. Das Implantat eignet sich jedoch auch zur Verwendung als Bohrloch-Abdeckung oder als Sternum-Verschluss. Bei der zuletzt genannten Verwendung können dem Fachmann allgemein bekannte Modifikationen vorgenommen werden. In diesem Zusammenhang sei allgemein auf das Dokument US 4,802,477 verwiesen.

Das in Fig. 1 dargestellte Implantat 10 zur Festlegung eines Knochendeckels an einem Schädelknochen ist vollständig aus Kunststoff (beispielsweise PEEK) gefertigt. Insbesondere bei einer Verwendung des Implantats als Sternum-Verschluss kann eine erhöhte strukturelle Stabilität des Implantats und daher eine zumindest teilweise Fertigung aus Metall wünschenswert sein.

Wie in Fig. 1 dargestellt, umfasst das Implantat 10 ein erstes, unteres Anlageteil 12 sowie ein zweites, oberes Anlageteil 14, die mittels eines Kopplungselements in Gestalt eines bezüglich Biegebewegungen flexiblen Bandes 16 zugfest miteinander gekoppelt sind. Unter Zug wird hier eine Bewegung der beiden Anlageteile 12, 14 voneinander weg verstanden. Wie allgemein bekannt, wird zur Festlegung des Knochendeckels am Schädelknochen in einem ersten Schritt das Band 16 in einem Spalt zwischen dem Knochendeckel und dem Schädelknochen angeordnet. In einem zweiten Schritt wird dann das obere Anlageteil 14 so lange entweder von Hand oder mittels eines Applikationsinstruments entlang des Bandes 16 in Richtung auf das untere Anlageteil 12 verschoben, bis der Knochendeckel und der Schädelknochen sicher zwischen den beiden Anlageteilen 12, 14 eingeklemmt sind. In der Regel finden insgesamt zwischen zwei und sechs solcher Implantate 10 Verwendung, die entlang des Außenumfangs des Knochendeckels gleichmäßig voneinander beabstandet und ggf. im Bereich von Bohrlöchern angeordnet werden.

Im Ausführungsbeispiel bilden das untere Anlageteil 12 und das Band 16 eine einstückige, mittels eines Spritzguss-Verfahrens gefertigte erste Baugruppe. Der Anspritzpunkt am Band 16 ist mit dem Bezugszeichen 22 gekennzeichnet. Das auf das Band 16 aufgefädelte obere Anlageteil 14 bildet eine zweite Baugruppe aus einem Anlageteil-Grundkörper 18 sowie einem im Grundkörper 18 befestigten Einsatz 20.

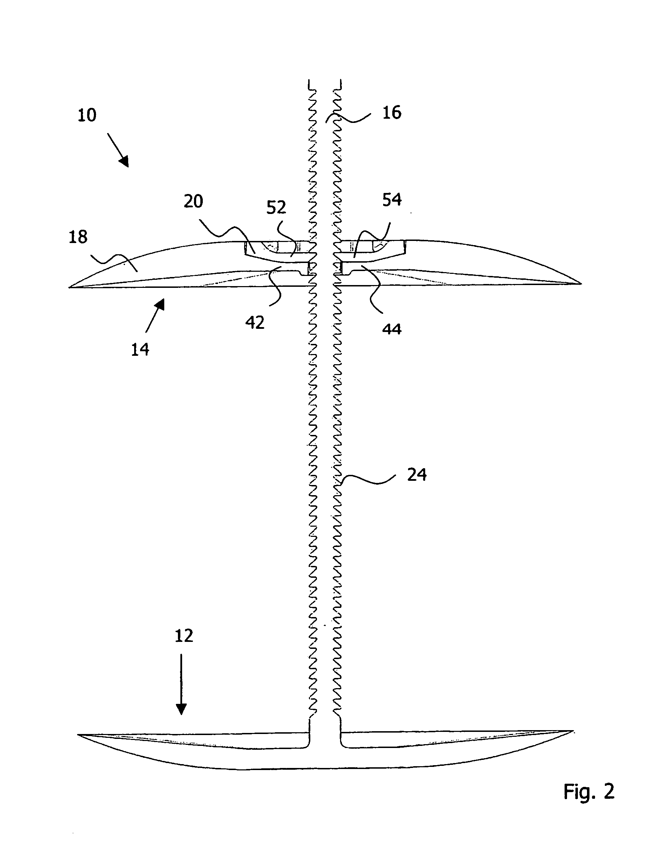

Fig. 2 zeigt eine Schnittdarstellung des unteren Teils des Implantats 10 in einer zur langen Seite des Bandes 16 senkrechten Ebene. In Fig. 2 deutlich zu erkennen ist die gekrümmte Grundform der beiden Anlageteile 12, 14. Jedes der beiden Anlageteile 12, 14 ist in Richtung auf das jeweils gegenüber liegende Anlageteil 14, 12 konkav gekrümmt. Die Starrheit bzw. die Verformbarkeit des für die Anlageteile 12, 14 konkret zum Einsatz gelangenden Materials (bzw. der entsprechenden Materialmischung) kann derart gewählt sein, dass sich im Rahmen des Festlegens des Implantats 10 an den Knochenteilen die zentralen Abschnitte der Anlageteile 12, 14 elastisch aufeinander zu verformen.

Die dem Anlageteil 14 zugewandte Seite des Anlageteils 12 und/oder die dem Anlageteil 12 zugewandte Oberfläche des Anlageteils 14 kann aufgeraut sein. Die Rautiefe kann sich im Bereich zwischen einem und 100 µm bewegen. Bevorzugt ist eine Rautiefe zwischen fünf und 30 µm, beispielsweise gemäß Erodierstruktur VDI 3400 Ref. 45 (entsprechend einer Rautiefe von 18 µm). Eine derartige Aufrauung verhindert ein Verrutschen des Implantats im Rahmen des Festlege-Vorgangs und auch im implantierten Zustand.

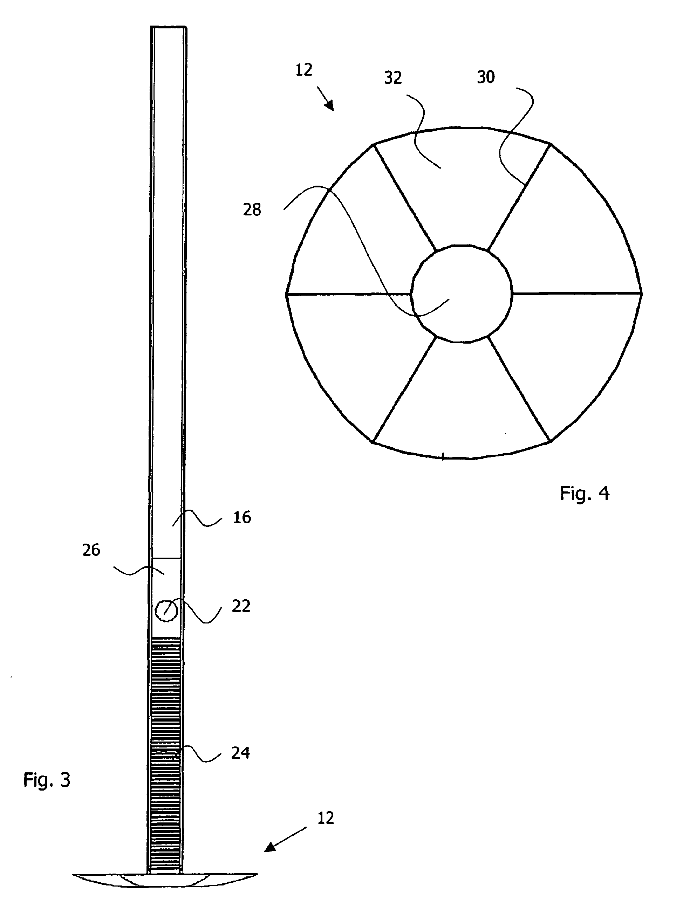

Fig. 3 zeigt eine Seitenansicht der einstückig gefertigten Baugruppe aus unterem Anlageteil 12 und Band 16. Gut zu erkennen ist der Sachverhalt, dass sich eine als Zahnung 24 ausgestaltete Oberflächenkonturierung des Bandes 16 ausgehend vom unteren Anlageteil 12 über weniger als ungefähr ein Drittel der Gesamtlänge des Bandes 16 erstreckt. Die verbleibende Länge des Bandes 16 ist im Wesentlichen glatt oder angeraut und kann als Angriffspunkt für ein Applikationsinstrument dienen.

Solche Applikationsinstrumente sind allgemein bekannt und werden verwendet, um das obere Anlageteil 14 in Richtung auf das untere Anlageteil 12 zu schieben. Manche Applikationsinstrumente sind darüber hinaus ausgebildet, um einen über das obere Anlageteil 14 abstehenden Abschnitt des Bandes 16 abzuschneiden.

Das Band 16 besitzt im Bereich seines glatten oder aufgerauten Abschnitts eine erste Stärke, welche in einem Übergangsbereich 26 allmählich zunimmt bis zu einer zweiten, etwas größeren Stärke im Bereich der Zahnung 24. Dieser Verlauf der Materialstärke vereinfacht das Auffädeln des oberen Anlageteils 14 auf das Band 16.

Fig. 4 zeigt die Baugruppe aus Fig. 2 (und insbesondere das untere Anlageteil 12) von unten. Zu erkennen ist die ungefähr kreisrunde Außenkontur des unteren Anlageteils 12. Das untere Anlageteil 12 besitzt einen zentralen, kreisrunden Abschnitt 28 maximaler Materialstärke, der als Verankerungsbereich für das Band 16 (das sich in Fig. 3 in die Zeichenebene hinein erstreckt) dient.

Ausgehend von dem zentralen Abschnitt 28 sind insgesamt sechs Rippen 30 vorgesehen. Die Rippen 30 erstrecken sich vom zentralen Abschnitt 28 in radialer Richtung bis zum Außenumfang des unteren Anlegeteils 12. Die Rippen 30 besitzen eine Verstärkungsfunktion im Bezug auf die strukturelle Stabilität des unteren Anlageteils 12 bei Beaufschlagung des Bandes 16 mit einer Zugkraft und dem damit einhergehenden in Anlage gelangen des Anlageteils 12 mit den zu koppelnden Knochenplatten.

Aufgrund der konkaven Grundform des unteren Anlageteils 12 besitzt jede der Rippen 30 eine ebenfalls konkave Krümmung entlang ihrer radialen Erstreckung. Zwei benachbarte Rippen 30 definieren jeweils ein Segment 32 mit einer an die Krümmung der Rippen 30 angenäherten Krümmung in radialer Richtung. Die Krümmung jedes Segments 32 in Umfangsrichtung (also senkrecht zur radialen Richtung) ist hingegen deutlich geringer.

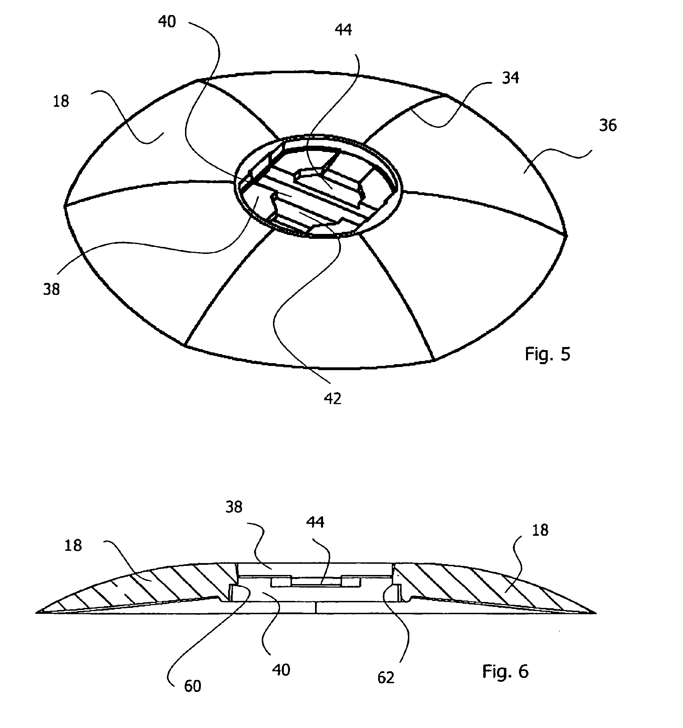

Fig. 5 zeigt in perspektivischer Ansicht den Grundkörper 18 des oberen Anlageteils. Die Formgebung des Grundkörpers 18 des oberen Anlageteils 14 einschließlich des Vorsehens von insgesamt sechs Rippen 34 mit dazwischen angeordneten Segmenten 36 entspricht der Grundform des oben im Zusammenhang mit Fig. 4 erläuterten unteren Anlageteils 12. Aus diesem Grund wird auf diese Merkmale des Grundkörpers 18 hier nicht näher eingegangen.

Deutlich zu erkennen in Fig. 5 ist eine mittig im Grundkörper 18 ausgebildete Vertiefung 38 zur Aufnahme des in den Fign. 1 und 2 dargestellten Einsatzes 20. Der Einsatz 20 selbst ist in Fig. 5 nicht dargestellt.

Die Vertiefung 38 mündet in eine rechteckige Öffnung 40 zur Aufnahme des Bandes 16 und von am Einsatz ausgebildeten Verbindungselementen. Aus Fig. 5 ersichtlich ist die Tatsache, dass in der Vertiefung 38 zwei versenkte Gegenlager 42, 44 vorgesehen sind, deren Funktion später noch näher erläutert werden wird.

Fig. 6 zeigt einen Schnitt durch den Grundkörper 18 in einer parallel zur Längsseite der Öffnung 40 verlaufenden und die Öffnung 40 beinhaltenden Ebene. Deutlich zu erkennen ist erneut die konkave Form des Grundkörpers 18 sowie das innerhalb der Vertiefung 38 ausgebildete versenkte Gegenlager 44.

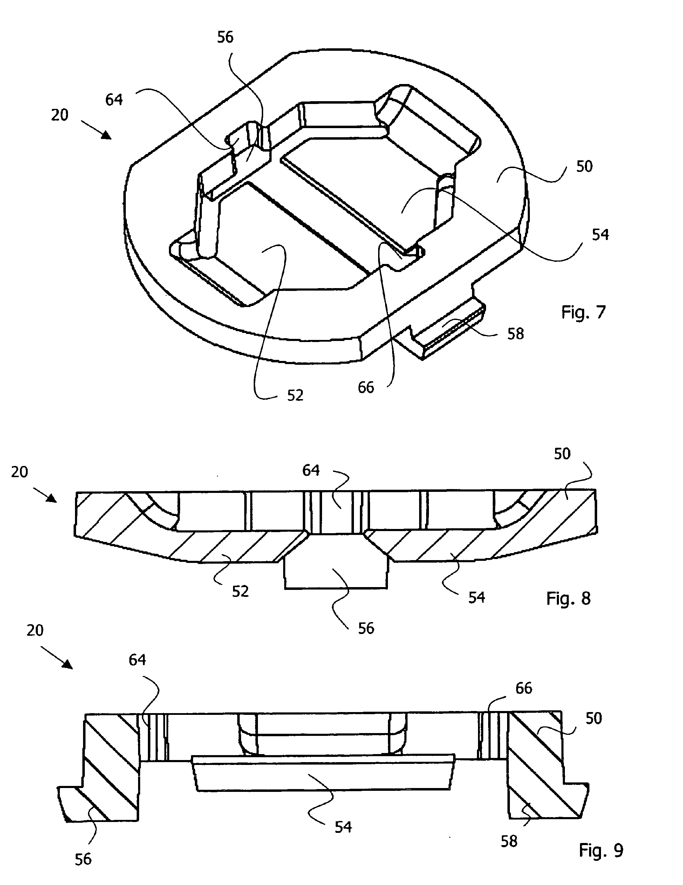

Fig. 7 zeigt eine perspektivische Ansicht des Einsatzes 20. Der Einsatz 20 besitzt einen Grundkörper 50, dessen Außenkontur der Innenkontur der Vertiefung 38 im Grundkörper 18 gemäß Fig. 5 entspricht. Der Einsatz 20 kann daher formschlüssig und drehfest in der Vertiefung 38 aufgenommen werden.

Am Grundkörper 50 sind zwei sich gegenüberliegende Festlege-Elemente in Gestalt auslenkbarer Zungen 52, 54 ausgebildet. Jede der beiden Zungen 52, 54 ist in einer parallel zum Band 16 verlaufenden Ebene nach oben sowie nach unten elastisch auslenkbar. Die einander zugewandten vorderen Enden der Zungen 52, 54 weisen voneinander einen Abstand auf, welcher in Abhängigkeit von der Stärke des gezahnten Bandabschnittes derart gewählt ist, dass die vorderen Enden der Zungen 52, 54, wie in Fig. 2, gezeigt mit der am Band 16 ausgebildeten Zahnung 24 zusammenwirken können.

Zur Erzielung eines Formschlusses zwischen den Zungen 52, 54 und der Zahnung 24 des Bandes 16 sind die vorderen Enden der Zungen 52, 54, wie in der Schnittdarstellung gemäß Fig. 8 erkennbar, spitz zulaufend ausgebildet. Diese Formgebung der vorderen Enden der Zungen 52, 54 entspricht der sägezahnartigen Profilierung der Zahnung 24. Die vorderen Enden der beiden Zungen 52, 54 können daher formschlüssig zwischen zwei benachbarten Zähnen 24 aufgenommen werden. Aufgrund der asymmetrischen Profilierung jedes der Zähne 24 erfolgt die Wechselwirkung zwischen den Zungen 52, 54 des Einsatzes 20 und den Zähnen 24 des Bandes 16 derart, dass einem Verschieben des oberen Anlageteils 14 in Richtung auf das untere Anlageteil 12 ein geringerer Wiederstand entgegengesetzt wird als einem Verschieben des oberen Anlageteils 14 in die umgekehrte Richtung.

Um nun den Widerstand, welchen die Wechselwirkung zwischen den Zungen 52, 54 und den Zähnen 24 einer Bewegung des oberen Anlageteils 14 vom unteren Anlageteil 12 weg entgegensetzt, (und damit die maximal erzielbare Klemmkraft) weiter zu erhöhen, gelangen die bereits oben im Zusammenhang mit Fig. 5 erläuterten, am Grundkörper 18 des oberen Anlageteils 14 ausgebildeten Gegenlager 42, 44 zum Einsatz. Wie in Fig. 2 dargestellt, liegen die beiden Zungen 52, 54 im Ausgangszustand flächig an den beiden Gegenlagern 42, 44 an. Die Gegenlager 42, 44 stützen daher die Zungen bei einer Bewegung des oberen Anlagenteils 14 vom unteren Anlageteil 12 weg ab. Dieses Abstützen wirkt einer Auslenkung der Zungen 52, 54 nach unten und der damit einhergehende Beweglichkeit des oberen Anlageteils 14 in Fig. 2 nach oben entgegen, da sich die vorderen Spitzen der Zungen 52, 54 nicht (oder nur unter hohem Kraftaufwand) aus der Zahnung 24 lösen können. Insgesamt erhöht die abstützende Wirkung der Gegenlager 42, 44 die maximal mit dem Implantat 10 erzielbare Klemmkraft deutlich gegenüber herkömmlichen Implantaten und insbesondere von Implantaten auf Kunststoffbasis.

Das Abstützen der Zungen 52, 54 an den Gegenlagern 42, 44 im Rahmen einer Krafteinleitung, welche die beiden Anlageteile 12, 14 voneinander weg drängt, erhöht natürlich drastisch die in den Einsatz 20 eingeleiteten Gegenkräfte. Diese Gegenkräfte sind darauf gerichtet, den Einsatz 20 aus dem Grundkörper 18 zu lösen. Aus diesem Grund ist eine effiziente Verankerung des Einsatzes 20 im Grundkörper 18 unerlässlich.

Wie in Fig. 7 dargestellt, umfasst der Grundkörper 50 des Einsatzes 20 zwei als Rastelemente 56, 58 ausgebildete Verbindungselemente zur Befestigung des Einsatzes 20 am Grundkörper 18 des oberen Anlageteils 14. Die beiden Rastelemente 56, 58 sind hakenförmig ausgebildet und an gegenüberliegenden Seiten des Grundkörpers 50 des Einsatzes 20 vorgesehen (vgl. Schnittdarstellung gemäß Fig. 9).

Der Einsatz 20 wird nun so in den Grundkörper 18 des oberen Anlageteils 14 eingesetzt, dass die Rastelemente 56, 58 den Grundkörper 18 des oberen Anlageteils 14 im Bereich der kurzen Seiten der Öffnung 40 hintergreifen. Bezug nehmend auf die Schnittdarstellung des Grundkörpers 18 gemäß Fig. 6 hintergreifen die hakenförmigen Ausformungen der Rastelemente 56, 58 den Grundkörper 18 also im Bereich der mit den Bezugszeichen 60 und 62 versehenen Stufen des Grundkörpers 18.

Die zum Festlegen einer Knochenplatte an einem Schädelknochen aufzubringenden Klemmkräfte können nun derart hoch werden, dass die resultierenden Gegenkräfte die Gefahr eines Entrastens der Rastelemente 56, 58 mit sich bringen. Um dieser Gefahr zu begegnen, sind die Rastelemente 56, 58 bezüglich einer Bewegung in eine Lösestellung (das heißt in Fig. 9 in Richtung auf die Zunge 54) am Band 16 abgestützt. Zu diesem Zweck weist der in Fig. 7 dargestellte Grundkörper 50 des Einsatzes 20 in an die Rückseiten der Rastelemente 56, 58 angrenzenden Bereichen Führungsrillen 64, 66 zur Führung des Bandes 16 entlang seiner kurzen Seiten auf. Dieser Sachverhalt ist auch in Fig. 1 dargestellt.

Im Ergebnis stützen sich die Rastelemente 56, 58 bei einer Bewegung in Richtung auf eine Lösestellung (in Fig. 9 also in Richtung auf die Zunge 54) an den kurzen Seiten des Bandes 16 ab. Dieses Abstützen wirkt einer weiteren Bewegung der Rastelemente 56, 58 in Richtung auf ihre Lösestellung entgegen, so dass der Einsatz 20 zuverlässig im Grundkörper 18 des oberen Anlageteils 14 verankert bleibt. Der darauf basierende Vorteil des Erzielens äußerst hoher Klemmkräfte wird durch die bereits oben erläuterte Formgebung der beiden Anlageteile 12, 14 mit versteifend wirkenden Rippen 30, 34 zuverlässig unterstützt.

Obwohl die Versteifungsrippen 32, 34, die Gegenlager 42, 44 und das Abstützen der Rastelemente 56, 58 in ihrer Kombination besonders vorteilhafte Effekte bewirken, lassen sich diese technischen Merkmale auch unabhängig voneinander einsetzen, um die mit dem jeweiligen Merkmal in Zusammenhang stehenden technischen Effekte einzeln zu erzielen. Außerdem ist darauf hinzuweisen, dass sich die technischen Merkmale zwar besonders vorteilhaft in Kombination mit dem Werkstoff Kunststoff auswirken, das Implantat aber auch teilweise oder vollständig und unter Ausnutzung der selben technischen Effekte aus Metall gefertigt werden kann.

## Patentansprüche

1. Implantat (10) zur Verwendung für benachbart zueinander angeordnete Knochenplatten, mit:
- einem ersten Anlageteil (12);
- einem zweiten Anlageteil (14), das einen Grundkörper (18) und einen Einsatz (20) aufweist, wobei wenigstens ein auslenkbares Festlege-Element (52, 54) entweder am Grundkörper (18) oder am Einsatz (20) ausgebildet ist; und
- einem sich durch das zweite Anlageteil (14) hindurch erstreckenden und mit dem Festlege-Element (52, 54) zusammenwirkenden Kopplungselement (16) zur zugfesten Kopplung des ersten Anlageteils (12) mit dem zweiten Anlageteil (14), wobei das Kopplungselement (16) in einen Spalt zwischen den Knochenplatten derart einfügbar ist, dass die beiden Knochenplatten zwischen den beiden Anlageteilen (12, 14) angeordnet sind,
wobei ein Gegenlager (42, 44) für das Festlege-Element (52, 54) an der anderen Komponente von Einsatz (20) und Grundkörper (18) ausgebildet ist, so dass das Gegenlager (42, 44) das Festlege-Element (52, 54) bei einer Bewegung des zweiten Anlageteils (14) vom ersten Anlageteil (12) weg abzustützen vermag,
**dadurch gekennzeichnet dass** der Einsatz am Grundkörper befestigt ist.

2. Implantat nach Anspruch 1, **dadurch gekennzeichnet,**
**dass** das zweite Anlageteil (14) wenigstens zwei sich bezüglich des Kopplungselements (16) gegenüber liegende Festlege-Elemente (52, 54) aufweist und ein Gegenlager (42, 44) für jedes der Festlege-Elemente (52, 54) vorgesehen ist.

3. Implantat nach einem der Ansprüche 1 oder 2, **dadurch gekennzeichnet, dass** das wenigstens eine Festlege-Element (52, 54) als Zunge oder Klaue ausgebildet ist.

4. Implantat nach einem der Ansprüche 1 bis 3, **dadurch gekennzeichnet, dass** das Kopplungselement (16) eine Oberflächenkonturierung (24) aufweist und das wenigstens eine Festlege-Element (52, 54) zum zugfesten Festlegen des Kopplungselements (16) am zweiten Anlageteil (14) mit der Oberflächenkonturierung (24) zusammenwirkt, wobei die Oberflächenkonturierung (24) insbesondere als eine Abfolge von Zähnen ausgebildet ist, vorzugsweise dahingehend, dass die Zähne ein asymmetrisches Profil derart aufweisen, dass einer Bewegung des zweiten Anlageteils (14) auf das erste Anlageteil (12) zu ein geringerer Widerstand entgegengesetzt ist als einer Bewegung in der umgekehrten Richtung.

5. Implantat nach einem der Ansprüche 1 bis 4, **dadurch gekennzeichnet, dass** der Einsatz (20) mittels wenigstens eines Verbindungselements (56, 58) mit dem Grundkörper (18) verbunden ist und das wenigstens eine Verbindungselement (56, 58) sich bezüglich einer Bewegung in eine Lösestellung des Verbindungselementes (56, 58) am Kopplungselement (16) abstützt.

6. Implantat nach Anspruch 5, **dadurch gekennzeichnet,**
**dass** das wenigstens eine Verbindungselement (56, 58) am Grundkörper (18) und/oder am Einsatz (20) ausgebildet ist.

7. Implantat nach einem der Ansprüche 5 und 6, **dadurch gekennzeichnet, dass** der Einsatz (20) mittels zweier Verbindungselemente (56, 58) am Grundkörper (18) befestigt ist, die sich bezüglich einer Bewegung in eine Lösestellung des Verbindungselements (56, 58) an gegenüberliegenden Seiten des Kopplungselements (16) abstützen.

8. Implantat nach einem der Ansprüche 5 bis 7, **dadurch gekennzeichnet, dass** das Verbindungselement (56, 58) als Rastelement ausgebildet ist und/oder der Einsatz wenigstens ein Festlege-Element (52, 54) trägt, das dazu ausgebildet ist, das zweite Anlageteil (14) am Kopplungselement (16) festzulegen.

9. Implantat (10) nach einem der Ansprüche 1 bis 8 **dadurch gekennzeichnet, dass** das wenigstens eine konkav gekrümmte Anlageteil (12, 14) eine Mehrzahl von Rippen (30, 34) aufweist, die sich ausgehend von einem zentralen Abschnitt (28, 38) dieses Anlageteils (12, 14) radial nach außen erstrecken.

10. Implantat nach Anspruch 9, **dadurch gekennzeichnet,**
**dass** zwischen vier und zehn Rippen (30, 34) vorgesehen sind.

11. Implantat nach Anspruch 9 oder 10, **dadurch gekennzeichnet,**
**dass** jeweils zwei benachbarte Rippen (30, 34) ein Anlageteil-Segment (32, 36) definieren und jedes Segment (32, 36) eine Krümmung im Wesentlichen nur in radialer Richtung aufweist und/oder dass beide Anlageteile (12, 14) eine konkave Krümmung und Rippen (30, 34) aufweisen.

12. Implantat nach einem der Ansprüche 9 bis 11, **dadurch gekennzeichnet, dass** wenigstens eines der beiden Anlageteile (12, 14) aus Kunststoff gefertigt ist, wobei vorzugsweise das Implantat (10) wenigstens weitestgehend aus PEEK gefertigt ist.

13. Implantat nach einem der Ansprüche 9 bis 12, **dadurch gekennzeichnet, dass** das erste Anlageteil (12) einstückig mit dem Kopplungselement (16) gefertigt ist und/oder dass das erste Anlageteil (12) auf seiner dem zweiten Anlageteil (14) zugewandten Oberfläche und/oder das zweite Anlageteil (14) auf seiner dem ersten Anlageteil (12) zugewandten Oberfläche aufgeraut ist.

## Claims

1. An implant (10) for use for adjacently arranged bone plates, having:
- a first bearing part (12);
- a second bearing part (14), which comprises a main body (18) and an insert (20), wherein at least one deflectable fixing element (52, 54) is provided either on the main body (18) or on the insert (20); and
- a coupling element (16) extending through the second bearing part (14) and cooperating with the fixing element (52, 54) for tension-proof coupling of the first bearing part (12) with the second bearing part (14), wherein the coupling element (16) is insertable into a gap between the bone plates in such a way that the two bone plates are arranged between the two bearing parts (12, 14),
wherein a counter support (42, 44) for the fixing element (52, 54) is provided on the other one of the insert (20) and main body (18), such that the counter support (42, 44) is capable of supporting the fixing element (52, 54) in the event of movement of the second bearing part (14) away from the first bearing part (12), **characterized in that** the insert is attached to the main body.

2. The implant according to claim 1, **characterized in that**
the second bearing part (14) has at least two fixing elements (52, 54) opposite one another with regard to the coupling element (16), and a counter support (42, 44) is provided for each of the fixing elements (52, 54).

3. The implant according to one of claims 1 or 2, **characterized in that**
the at least one fixing element (52, 54) takes the form of a tongue or claw.

4. The implant according to one of claims 1 to 3, **characterized in that**
the coupling element (16) comprises a surface contouring (24) and the at least one fixing element (52, 54) cooperates with the surface contouring (24) for tension-proof fixing of the coupling element (16) to the second bearing part (14), wherein the surface contouring (24) particularly takes the form of a series of teeth, preferably so that the teeth have an asymmetrical profile, such that there is less resistance to movement of the second bearing part (14) towards the first bearing part (12) than to movement in the opposite direction.

5. The implant according to one of claims 1 to 4, **characterized in that**
the insert (20) is connected to the main body (18) by means of at least one connecting element (56, 58) and the at least one connecting element (56, 58) is supported against the coupling element (16) with regard to movement into a release position of the connecting element (56, 58).

6. The implant according to claim 5, **characterized in that**
the at least one connecting element (56, 58) is provided on the main body (18) and/or on the insert (20).

7. The implant according to one of claims 5 and 6, **characterized in that**
the insert (20) is attached to the main body (18) by means of two connecting elements (56, 58), which are supported against opposing sides of the coupling element (16) with regard to movement into a release position of the connecting element (56, 58).

8. The implant according to one of claims 5 to 7, **characterized in that**
the connecting element (56, 58) takes the form of a latching element and/or the insert bears at least one fixing element (52, 54) which is configured to fix the second bearing part (14) to the coupling element (16).

9. The implant (10) according to one of claims 1 to 8, **characterized in that**
the at least one concavely curved bearing part (12, 14) comprises a plurality of ribs (30, 34) extending radially outwards from a central portion (28, 38) of the bearing part (12, 14).

10. The implant according to claim 9, **characterized in that**
between four and ten ribs (30, 34) are provided.

11. The implant according to claim 9 or 10, **characterized in that**
two adjacent ribs (30, 34) in each case define a bearing part segment (32, 36) and each segment (32, 36) exhibits a curvature substantially in the radial direction only and/or that both bearing parts (12, 14) exhibit a concave curvature and ribs (30, 34).

12. The implant according to one of claims 9 to 11, **characterized in that**
at least one of the two bearing parts (12, 14) is made of plastics, wherein preferably the implant (10) is made at least substantially from PEEK.

13. The implant according to one of claims 9 to 12, **characterized in that**
the first bearing part (12) is made in one piece with the coupling element (16) and/or that the first bearing part (12) is roughened on its surface facing the second bearing part (14) and/or the second bearing part (14) is roughened on its surface facing the first bearing part (12).

## Revendications

1. Implant (10) destiné à être utilisé sur des plaques d'ostéosynthèse disposées contiguës l'une à l'autre, comprenant
- un premier élément d'appui (12),
- un deuxième élément d'appui (14) présentant un corps de base (18) et un insert (20), au moins un élément de serrage (52, 54) pouvant être dévié étant formé soit sur le corps de base (18) soit sur l'insert (20), et
- un élément d'accouplement (16) qui s'étend à travers le deuxième élément d'appui (14) et coopère avec l'élément de serrage (52, 54) pour permettre l'accouplement résistant à la traction du premier élément d'appui (12) au deuxième élément d'appui (14), ledit élément d'accouplement (16) pouvant être introduit dans un interstice entre les plaques d'ostéosynthèse de telle sorte que les deux plaques sont disposées entre les deux éléments d'appui (12, 14),
un contre-palier (42, 44) destiné à reposer contre l'élément de serrage (52, 54) étant formé sur l'autre composant de l'insert (20) et du corps de base (18) de manière à ce que l'élément de serrage (52, 54) puisse s'appuyer sur le contre-palier (42, 44) lorsque le deuxième élément d'appui (14) est déplacé et éloigné du premier élément d'appui (12),
**caractérisé en ce que** l'insert est fixé sur le corps de base.

2. Implant selon la revendication 1, **caractérisé en ce que**
le deuxième élément d'appui (14) présente au moins deux éléments de serrage (52, 54) disposés l'un en face de l'autre par rapport à l'élément d'accouplement (16) et qu'un contre-palier (42, 44) est prévu pour chacun des éléments de serrage (52, 54).

3. Implant selon la revendication 1 ou 2, **caractérisé en ce que**
ledit élément de serrage (52, 54) est réalisé sous forme de languette ou de griffe.

4. Implant selon l'une des revendications 1 à 3, **caractérisé en ce que**
l'élément d'accouplement (16) présente un contour de surface (24) et ledit élément de serrage (52, 54) coopère avec ce contour de surface (24) pour fixer de manière résistante à la traction l'élément d'accouplement (16) sur le deuxième élément d'appui (14), le contour de surface (24) étant en particulier réalisé sous la forme d'une succession de dents, préférentiellement de manière à ce que les dents présentent un profil asymétrique tel qu'il est opposé à un déplacement du deuxième élément d'appui (14) en direction du premier élément d'appui (12) une résistance plus faible que celle opposée à un déplacement en sens inverse.

5. Implant selon l'une des revendications 1 à 4, **caractérisé en ce que**
l'insert (20) est relié au moyen d'au moins un élément de raccordement (56, 58) au corps de base (18) et que cet élément de raccordement (56, 58) s'appuie sur l'élément d'accouplement (16) lors d'un déplacement dans une position de libération.

6. Implant selon la revendication 5, **caractérisé en ce que**
ledit élément de raccordement (56, 58) est formé sur le corps de base (18) et/ou sur l'insert (20).

7. Implant selon l'une des revendications 5 et 6, **caractérisé en ce que**
l'insert (20) est fixé sur le corps de base (18) au moyen de deux éléments de raccordement (56, 58) qui s'appuient sur les faces opposées de l'élément d'accouplement (16) lors d'un déplacement dans une position de libération.

8. Implant selon l'une des revendications 5 à 7, **caractérisé en ce que**
l'élément de raccordement (56, 58) est réalisé sous la forme d'un élément d'encliquetage et/ou l'insert supporte au moins un élément de serrage (52, 54) formé pour fixer le deuxième élément d'appui (14) sur l'élément d'accouplement (16).

9. Implant selon l'une des revendications 1 à 8, **caractérisé en ce que**
au moins un des éléments d'appui (12, 14) à courbure concave présente une pluralité de nervures (30, 34) qui s'étendent radialement vers l'extérieur en partant d'une partie centrale (28, 38) de cet élément d'appui (12, 14).

10. Implant selon la revendication 9, **caractérisé en ce que**
sont prévues entre quatre et dix nervures (30, 34).

11. Implant selon la revendication 9 ou 10, **caractérisé en ce que**
deux nervures contiguës (30, 34) définissent à chaque fois un segment d'élément d'appui (32, 36) et que chaque segment (32, 36) présente une courbure n'allant pour l'essentiel que dans la direction radiale et/ou que deux éléments d'appui (12, 14) présentent une courbure concave et des nervures (30, 34).

12. Implant selon l'une des revendications 9 à 11, **caractérisé en ce que**
au moins un des deux éléments d'appui (12, 14) est fabriqué à partir de matière synthétique, l'implant (10) étant préférentiellement fabriqué, au moins pour une très large part, à partir de PEEK.

13. Implant selon l'une des revendications 9 à 12, **caractérisé en ce que**
le premier élément d'appui (12) est fabriqué d'un seul tenant avec l'élément d'accouplement (16) et/ou que le premier élément d'appui (12) est grenaillé sur sa surface tournée vers le deuxième élément d'appui (14) et/ou que le deuxième élément d'appui (14) est grenaillé sur sa surface tournée vers le premier élément d'appui (12).
